# EUROPEAN PATENT SPECIFICATION

(11) **EP 0 446 948 B1**
(45) Date of publication and mention of the grant of the patent: **19.06.1996**
(21) Application number: 91104054.1
(22) Date of filing: 15.03.1991
(51) Int. Cl.: C12N 11/04

(54) **Biocatalysts immobilized by entrapment and process for their preparation**
Durch Einschliessen immobilisierte Biokatalysatoren und Verfahren zu ihrer Herstellung
Biocatalyseurs immobilisés par piégeage et leur procédé de préparation

(30) Priority: 15.03.1990 JP 62593/90
(43) Date of publication of application: 18.09.1991
(73) Proprietor: MITSUBISHI CHEMICAL CORPORATION, Chiyoda-ku, Tokyo 100 (JP)
(72) Inventor: Yoneyama, Takahiro, Matsudo-shi, Chiba-ken (JP); Yamagata, Hisashi, Tagu-cho, Ushiku-shi, Ibaraki-ken (JP); Shimazu, Mitsunobu, Inashiki-gun, Ibaraki-ken (JP); Odagiri, Masaki, Ushiku-shi, Ibaraki-ken (JP); Imanari, Makoto, Inashiki-gun, Ibaraki-ken (JP)
(74) Representative: Kraus, Walter, Dr.

(56) References cited:
- GB-A- 2 082 591
- GB-A- 2 119 737
- BIOTECHNOLOGY LETTERS, vol. 3, no. 8, 1981, GB; S. BIRNBAUM et al., pp. 393-400 "Covalent Stabilization of Alginate Gel for the Entrapment of Living Whole Cells"
- BIOTECHNOLOGY ABSTRACTS DATABASE, Derwent Publications Ltd., London (GB); abstract no. 8612266; A.B. SALLEH et al.:"Stabilization of Alginate Gel - for use as immobilization support"
- WORLD PATENT INDEX LATEST , Derwent Publications, Ltd., LONDON, GB; DATABASE WPIL, Accession No. 84-143230/23,& JP-A-59074984
- PATENT ABSTRACTS OF JAPAN, vol. 8, no. 232 (C- 248)(1669), 25 October 1984; & JP-A-59113889
- PATENT ABSTRACTS OF JAPAN, vol. 13, no. 372 (C- 627)(3720), 17 August 1989; JP-A-01128787
- BIOTECHNOLOGY & BIOENGINEERING, vol. 32, no. 6, 09 September 1987, pages 756-759, US; E. KOKUFUTA et al.:"Use of polyelectrolyte complex-stabilized calcium alginate gel for entrapment of beta-amylase"

## Description

The present invention relates to a process for preparing an immobilized biocatalyst having a great chemical strength by entrapping microbial cells or their treated substances with water-insoluble polyuronic acid.

Where enzymes or microorganisms are applied to the production of foods, drugs, etc. as the biocatalyst, it is industrially preferable to make their reuse or continuous use possible by immobilizing them. Many immobilization methods have been hitherto developed and they are roughly classified into the following three, i.e. a carrier binding method, a crosslinking method and an entrapping method.

Among these the entrapping method into the gel matrix of a polyuronic acid polyvalent metal salt like calcium alginate is generally less in the inactivation of enzyme activity involved by the immobilization of a biocatalyst partly because an enzyme and a gel are not bound together directly and partly because reaction conditions at the time of the immobilization are gentle. Thus, this is considered to be one of the most useful immobilization methods.

However, this method has such problem that in a reaction liquid free from polyvalent metal ions forming a gel such as calcium ions or aluminum ions in the pH over about 6 or in a reaction liquid containing ions such as sodium ions, phosphate ions or sulfate ions the gel is swollen slowly, in consequence of which biocatalysts begin to be leaked from the gel and become unfit for long-term, continuous use. This is attributable to such phenomenon that calcium ions crosslinking carboxyl anions of alginic acid will drop out under the above conditions.

In order to avoid such problems caused by the gel swelling the following reaction system and gel improvement method have been tried. That is:
1) A method by which calcium ions are added into a reaction liquid [e.g. Appl. Microbio. Biotechnol., 25, 186 (1986)].
2) A method by which an inorganic substance like sparingly soluble calcium salt is mixed in the gel [e.g. Japanese Laid-Open (kokai) Publn. No. 63-160584].
3) A method by which the gel is coated with a polycationic high polymer like polyethyleneimine [e.g. Biotechnol. Bioeng., 26, 1393 (1984)].
4) A method by which the gel is coated with glutaraldehyde or glutaraldehyde and hexamethylenediamine [e.g. Biotechnol. Bioeng., 21, 1697 (1979)].

However, the above method of 1) has such demerit that an adverse effect of calcium ions is made on the separation step, purification step, etc. at the time of its industrial working. Moreover, the methods of 2) and 3) have such disadvantage that since they do not essentially differ in the ion crosslinked structure of the gel, the gel swelling by the drop-out of crosslinked ions is inevitable, more or less. And the method of 4) has such weak point that the crosslinking of an aldehyde group is a crosslinking in which the clevage occurs simply depending on the effect of pH or the like.

S. Birnbaum et al., 1981, Biotechnology Letters, Vol. 3, No. 8, pages 393-400, discloses microbial cells (Saccharomyces cerevisiae) immobilized by entrapment in a gel of water-insoluble polyuronic acid (alginate gel) containing a crosslinking agent (polyethyleneimine) which is a water-soluble high polymer compound with functional groups that bind to the carboxyl groups of said polyuronic acid via amide and/or ester bonds and thereby crosslinking said polyuronic acid. In order to obtain said immobilized biocatalyst, three different methods are described which covalently stabilize the previously known alginate gels. Methods I and II comprise contacting the gel of water-insoluble polyuronic acid (alginate gel) entrapping the microbial cells with the crosslinking agent (polyethyleneimine) having the functional groups and in the presence of a condensation agent, either the glutaraldehyde of method I or the carbodiimide of method II. The disclosed stabilizations of alginate gels provide beads with entrapped biocatalysts having a high retention of their biological activity and a great stability upon contact with complexing anions (phosphate buffers).

The alginate gel beads of method I of the above-mentioned document are formed by subjecting polyethyleneimine and glutaraldehyde to a covalent bonding and then making the resulting covalent bond ionically bind to alginate gel polymer (compare page 394, lines 43-48, page 395, Figure 1 and page 395, line 20 to page 396, line 4). The alginate gel beads prepared according to method I differ therefore from the alginate beads prepared according to this invention.

In the process for the entrapment of living whole cells mentioned in method II of the above-mentioned document, as it is clearly seen from page 393, Summary, lines 4-6, page 395, lines 1-12, page 395, Figure 1, method II, "alginate sol was treated with carbodiimide and N-hydroxysuccinimide (to form active ester), mixed with cells and extruded into calcium solution. After rinsing the beads with water the beads were incubated in polyethyleneimine."

Therefore, according to method II of the above-mentioned document calcium-alginate-active ester gel beads are subjected to a reaction with a polyethyleneimine (crosslinking agent) after washing with water, that is, after inactive urea derivatives etc., which are reaction residues of carbodiimide, are removed by washing.

On the other hand, according to the present invention gel beads of polyuronic acid are subjected to a reaction with a crosslinking agent in the presence of a condensation agent. The process according to this invention is not disclosed or suggested by the above-mentioned reference.

It is an object of the present invention to provide a process for preparing biocatalysts immobilized by entrapment of the type which is small in the effect of pH on the reaction system and which is usable in a wide pH range and a process for preparing biocatalysts immobilized by entrapment of the type which is difficult to swell even where polyvalent metal ions forming a gel such as calcium ions or aluminum ions do not substantially exist in a reaction system or where ions dissolving a gel such as sodium ions, ammonium ions or phosphoric acid ions exist in such reaction system. Another object of the invention is to provide a process for preparing biocatalysts immobilized by entrapment of the type which is sufficiently fit for a long-term, continuous use. A further object of the invention is to provide a process for preparing biocatalysts immobilized by entrapment of the type which is less in the reduction of activity of the immobilized biocatalyst involved by immobilization.

Under these circumstances, the Inventors discovered that the above objects of the present invention would be attainable by crosslinking a polyuronic acid via an amide bond and/or an ester bond.

The present invention relates to a process for the preparation of an immobilized biocatalyst which comprises the following steps:
(i) mixing an aqueous solution of polyuronic acid with microbial cells or products derived therefrom,
(ii) adding dropwise the mixture obtained in the step (i) into an aqueous gelation solution to form a gel of water-insoluble polyuronic acid entrapping microbial cells or products derived therefrom, and
(iii) contacting the gel with a crosslinking agent containing in its molecule at least two of such reactive functional groups as are selected from the group consisting of an amino group and a hydroxyl group, in an aqueous medium in the presence of a condensation agent.

In the following section the present invention will be explained in more detail.

The polyuronic acid used in the present invention is usually used as a gel for the immobilization of microorganisms by entrapment.

Examples include an alginic acid, a pectic acid or a mixture thereof.

The crosslinking agent used to crosslink a polyuronic acid in the present invention is the one which contains in its molecule two or more functional groups capable of forming an amide bond and/or an ester bond by the reaction with a carboxyl group of the polyuronic acid. Examples of such functional group include an amino group, a hydroxy group or a functional derivative group thereof.

The crosslinking agent used here is preferable if being water-soluble per se. Especially preferable is a water-soluble high polymer compound.

Specific examples of the crosslinking agent may include a monomeric compound such as ethylene diamine, ethylene glycol, glycerol, ethanolamine, diethanolamine or triethanolamine and a high polymer compound such as polyvinyl alcohol, polyalkyleneimine (e.g. polyethyleneimine, polypropyleneimine, polybuthyleneimine, etc.), polyallylamine or polyvinylamine. Among these, the high polymer compound is desirable. Above all, polyethyleneimine or polyallylamine is especially preferable in view of the accessibility and the reactivity.

A mean molecular weight of the high polymer compound is not restricted, in particular. However, a range of about 10,000 to about 100,000 is advantageous in case of the polyvinyl alcohol and a range of about 300 to about 100,000, preferably about 1,000 to about 70,000 is advantageous in case of the polyalkyleneimine. Further, a range of about 5,000 to about 100,000 is advantageous in case of the polyallylamine. And a range of about 10,000 to about 50,000 is advantageous in case of the polyvinylamine.

While, as the microorganism to be immobilized according to the process of the present invention are applicable all of bacteria, yeasts, molds, ray fungus, etc. Their treated substances can be also used.

The treated substance here indicates all those which are obtainable by the cultivation of a destructed substance of the cell, its pulverized substance, its self-digested substance, etc. Further, the treated substance includes those obtainable by the agglutination or preliminary immobilization of the cell or its destructed substance.

Moreover, the present invention can be applied to the immobilization of an animal cell or a plant cell by entrapment in addition to the above microorganisms. These animal and plant cells are good even if they are not only naturally occurring cells but also those obtained by any artificial means such as genetic recombination technique or cell fusion technique, e.g. hybridoma.

The biocatalysts immobilized by entrapment can be formed by reacting a gel of the water-insoluble polyuronic acid containing microbial cells or their treated substances and the above crosslinking agent in an aqueous medium in the presence of a condensation agent.

As the condensation agent used at the moment can be given a water-soluble carbodiimide reagent as a preferable example [J. Org. Chem. 21, 439 (1956)]. Its concrete examples may include N-(3-dimethylamino)propyl-N'-ethylcarbodiimide hydrochloride. This compound is commercially available by Aldrich Chemical co., Inc., etc. This is easily accessible and preferable.

In the preparation of the instantly claimed biocatalysts immoblized by entrapment is prepared firstly suspension of the biocatalysts, microbial cells or their treated substances, being suspended uniformly in an aqueous solution of a water-soluble polyuronic acid compound such as sodium alginate, calcium alginate or ammonium or pectic acid.

The composition of this suspension here is preferably such as containing the polyuronic acid compound in a concentration of 5-100 g/l, especially 10-50 g/l, the biocatalyst in a concentration of 10-900 g (wet weight)/l, especially 50-200 g (wet weight)/l. Next, it is suitable that the immobilization and crosslinking using this suspension be conducted by following any one of the procedures below. That is:
(1) a process in which a water-insoluble gel is obtained in the way known per se which comprises adding dropwise a suspension to an aqueous gelation solution containing calcium ions, aluminum ions or the like, discharging the mixture, wetting it, etc. and then this gel is thrown into an aqueous mixture of the crosslinking agent and condensation agent, followed by allowing the mixture to stand or stirring it;
(2) a process in which a crosslinking agent having a concentration of 1-200 g/l, preferably 10-50 g/l is added to suspension and then a water-insoluble gel is obtained in the known way as mentioned above and then this gel is thrown into an aqueous solution of a condensation agent or an aqueous mixture of such a condensation agent and a crosslinking agent, followed by allowing the mixture to stand or stirring it; and
(3) a process in which a crosslinking agent and a condensation agent are added to an aqueous gelation solution and a suspension is added dropwise to this and the mixture is discharged, wetted, etc., followed by allowing it to stand or stirring it.

Among them the process of (1) and the process of (2) are preferable. In particular, the process of (1) is preferable.

The pH value of a treating solution used in the above gelation and crosslinking treatment is preferably 2-12, especially preferably 5-8. The concentration of the crosslinking agent here is preferably 1-200 g/l, especially preferably 3-100 g/l. In case of the condensation agent it is desirable to employ this in a molar concentration of 0.1 to 5 times based on the number of moles of a carboxyl group in the used polyuronic acid compound. The treatment temperature and time are preferably 5-50° C and 1 to 100 hours, respectively. Further, the solvent used in the above treatment is preferably water but there is no problem even if using an organic solvent such as alcohol, acetone, acetonitrile, ether or dimethylformamide or a mixture thereof as required.

A biocatalyst immobilized by entrapment after the completion of the treatment can be used in an object catalystic reaction by recovering it via filtration and then washing it thoroughly in water.

The biocatalyst immobilized by entrapment has an excellent characteristic of being difficult to swell, as above. Concretely, the weight of the immobilized biocatalyst after being put into an aqueous 0.2 M disodium hydrogenphosphate solution in an amount of 100 times the weight of the biocatalyst and being shaked at 40° C for two hours is merely not more than double the original weight.

The shape of the biocatalyst immobilized by entrapment is not especially restricted and there is no problem even if the biocatalyst assumes any shape like being spherical, fibrous, filmy, cubic, etc. If the claimed biocatalyst is in the form of a fiber or a film, its size or thickness is 5 mm or less on the average, preferably 2 mm or less. If the biocatalyst assumes a spherical or cubic shape, its volume is 150 mm³ or less on the average, preferably 50 mm³ or less.

The immobilized biocatalyst is a gel entrapping Biocatalyst having a structure in which a carbonyl group of polyuronic acid is crosslinked with a covalent bond such as amide bond or ester bond. Compared with the conventional gels obtained by including a biocatalyst with a polyvalent metal salt of polyuronic acid, the swelling depending on the pH of a reaction liquid or the effect of ions in the reaction liquid is inhibited notably. Namely, said immobilized biocatalyst is extremely high in the chemical strength and has an effect of more amplifying an industrial use range of the gels of immobilized polyuronic acid.

### [Examples]

In the following section the present invention will be fully explained by way of the working examples but the present invention is not restricted by these examples.

### Example 1

Commercially available sodium alginate (viscosity: 800-1200 cps in 10 g/l of an aqueous solution at 20° C) was dissolved in a hydrothermal solution at 60° to 70° C), thereby to prepare 50 g/l of an aqueous solution. The so obtained aqueous solution was cooled until room temperature.

Successively, 60 ml of the above aqueous solution was added to 10 g (wet weight) of Pseudomonas putida (IFO 12996) containing amino acid racemase cultured in a culture medium (pH of 7.2) having the composition shown in Table 1 below at 30° C for 24 hours. Water was further added to the mixture to bring its total amount to 100 ml, followed by mixing them well.

This mixture was added dropwise to 400 ml of an aqueous 0.1 M calcium chloride solution and the mixture was stirred gently for additional two hours as such. There resulted 53.1 g (wet weight) of a spherical calcium alginate gel.

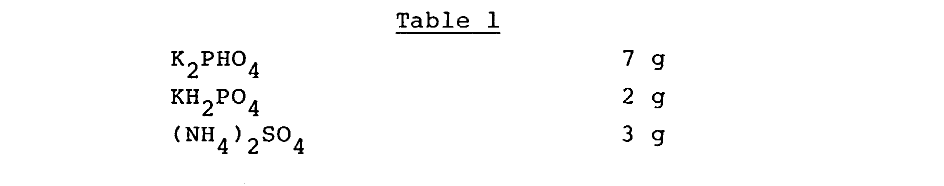

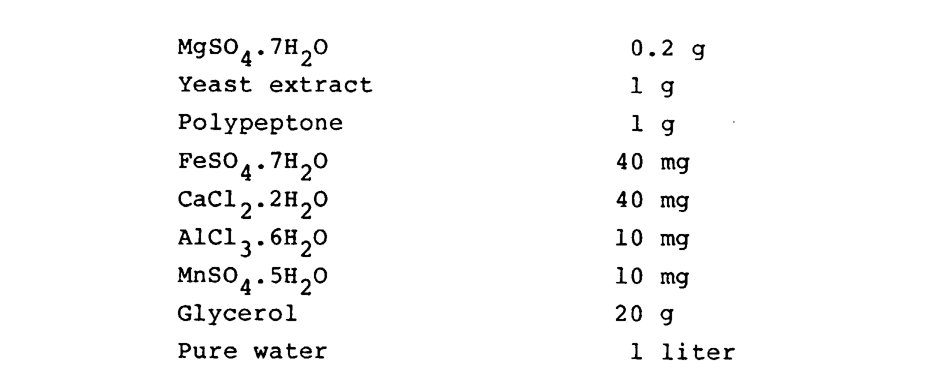

Next, crosslinking of the above gel was conducted in accordance with the following method.

That is, 10 g/l of an aqueous polyethyleneimine solution was neutralized with a concentrated sulfuric acid. Then 10.0 g of the above gel was added to 49 ml of a crosslinked solution obtained by adding to the above aqueous solution 550 mg of N-(3-dimethylamino)propyl-N'-ethylcarbodiimide hydrochloride. This was stirred gently at 23° C for 12 hours.

This was filtered/washed in water thereby to produce 7.0 g of an object immobilized microbial cell of Pseudomonas putida.

By the use of three kinds of immobilized microbial cells including the so obtained microbial cell their amino acid racemase activities were determined. As three kinds of immobilized microbial cells were used the following types (a) to (c):
(a) Immobilized microbial cell produced in said Example 1,
(b) Immobilized microbial cell which is merely immobilized with calcium chloride, and
(c) Immobilized microbial cell produced according to Example 1 except that no carbodiimide agent is used.

Into a 30 ml capacity conical flask were put 50 beads of each kind and 5 ml of an aqueous L-serine solution (0.1 mol/l) whose pH was adjusted to be 7.8 with caustic soda was added to this. This was shaked at 37° C for one hour. The degree of racemization of L-serine into D-serine in this interval was determined by means of a chiral column chromatography. The immobilized microbial cell after determination was washed in water for five minutes and the same activity determination was repeated three times. The result obtained is shown in Table 2.

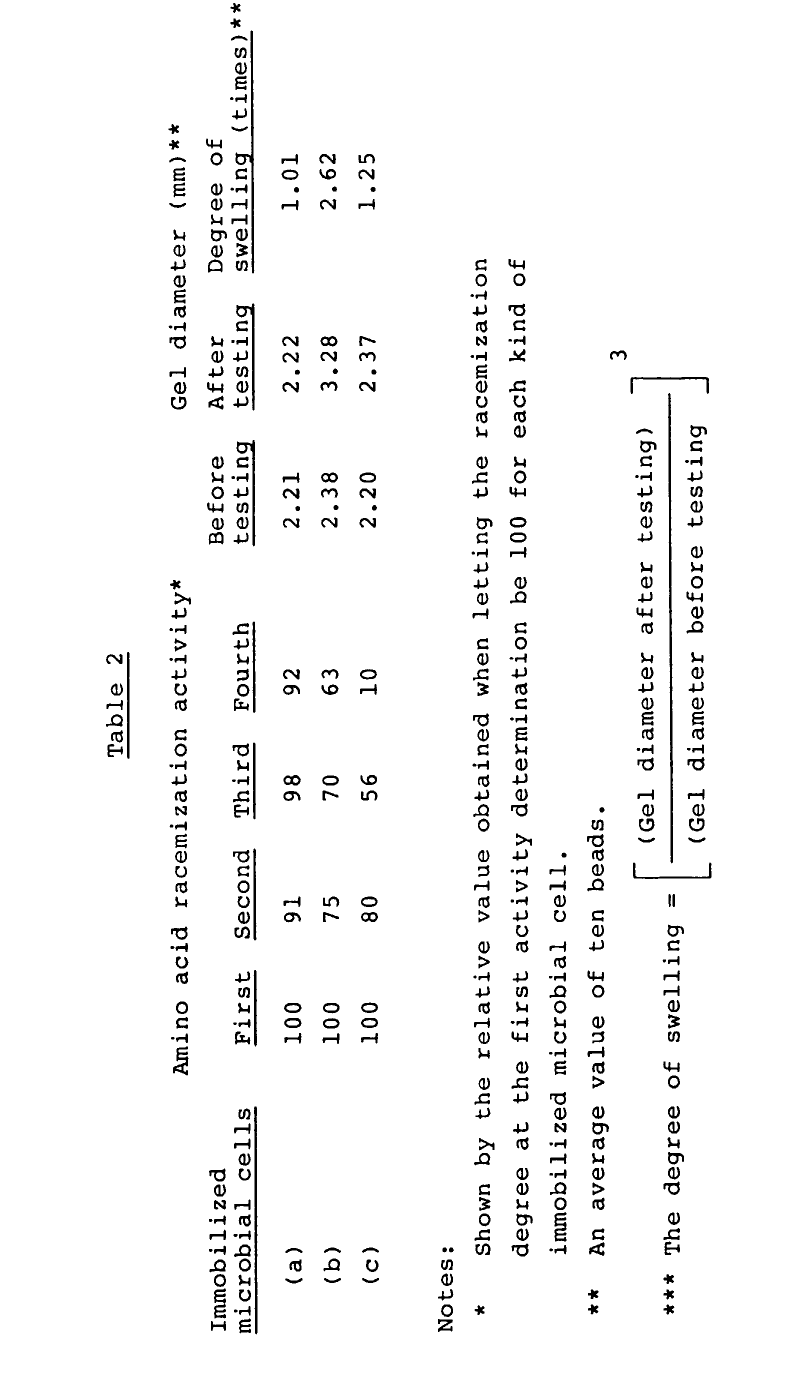

### Example 2

Three kinds of immobilized microbial cells including the above immobilized microbial cells were filled in the column thereby to determine its activity life at the time of the continuous reaction. As three kinds of immobilized microbial cells were used the following types (a) to (c):
(a) Immobilized microbial cell produced in said Example 1,
(b) Immobilized microbial cell which is merely immobilized with calcium chloride, and
(c) Immobilized microbial cell produced according to Example 1 except that no polyethyleneimine is used.
5 g of each kind of immobilized microbial cell was filled in a glass column having an inner diameter of 14 mm and a length of 12 cm (the height of filling was about 7 cm). An aqueous L-serine solution (0.15 mol/l) dissolved in 0.1 mol/l of a tris buffer solution was caused to pass through this column for seven days at 30° C in the up flow system of 20 ml/hr. The result obtained was shown in Table 3.

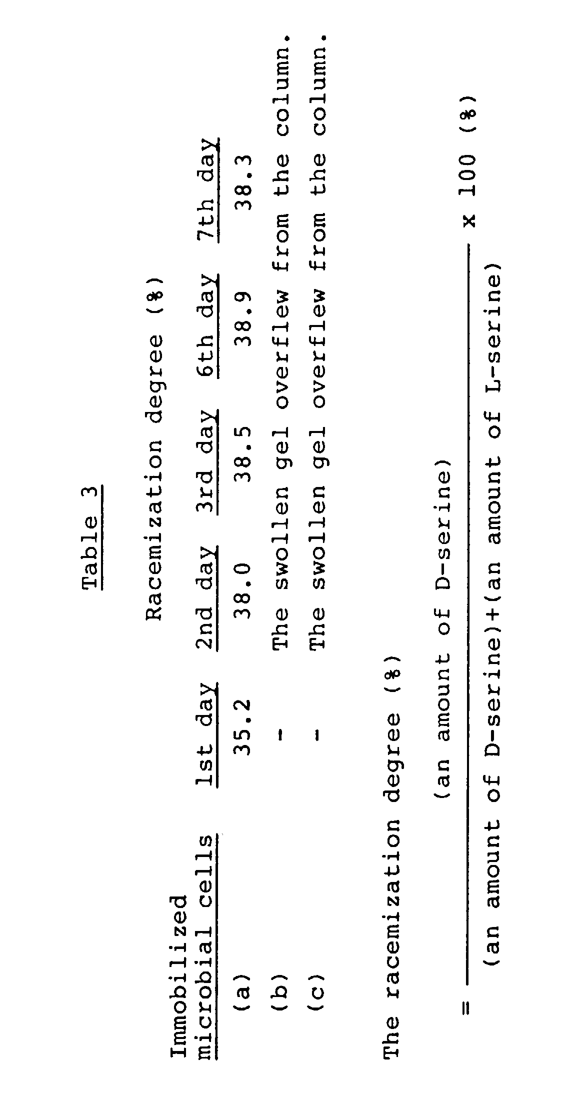

### Example 3

Escherichia coli K-12 YK 2009 (FERM BP-3244) was inoculated into a 500 ml capacity conical flask containing 100 ml of a culture medium having a composition shown in Table 4 and then cultured at 37° C for 24 hours. Moreover, 2 v/v % of said culture was inoculated into 100 ml of a culture medium having a composition shown in Table 5 and an indole acrylic acid was added to the culture medium in a concentration of 100 µg/ml. Its culturing by shaking was conducted at 37° C for 5 hours. To 5 g (wet weight) of the microbial cells collected centrifugally were added 20 g of a physiological saline solution and 25 g of an aqueous sodium alginate (50 g/l) prepared in Example 1, followed by mixing them well under ice-cooling.

**Table 4**

| | |
|---|---|
| K₂HPO₄ | 7.0 g |
| KH₂PO₄ | 2.0 g |
| (NH₄)₂SO₄ | 1.0 g |
| MgSO₄.7H₂O | 0.1 g |
| Glucose | 2.0 g |
| Water | 1 liter |

**Table 5**

| | |
|---|---|
| Tryptone | 10 g |
| Yeast extract | 5 g |
| NaCl | 5 g |
| Water | 1 liter |
| pH | 7.2 |

This was added dropwise to 50 ml of an aqueous 0.2 M calcium chloride solution at 10° C. After this dropwise addition the mixture was stirred gently for additional two hours thereby to prepare a spherical calcium alginate gel. While, a liquid of crosslinking agents having a composition shown in Table 6 was prepared under ice-cooling. Immediately after the preparation of the liquid of crosslinking agents the above gel was thrown into the liquid and shaked at 30° C for 50 hours. This was filtered and the residue was rinsed in a physiological saline solution thereby to prepare 12 g of immobilized microbial cell of Escherichia coli (average gel diameter of 1.2 mm).

**Table 6**

| | |
|---|---|
| An aquous 30 % polyethyleneimine solution (molecular weight of about 70,000) | 3.6 g |
| 6N hydrochloric acid | 2.6 g |
| N-(3-dimethylamino)propyl-N'-ethylcarbodiimide hydrochloride | 1.2 g |
| pH | 6.5 g |

Tryptophansyntase activity of this immobilized microbial cell of Escherichia coli was measured in accordance with the method like Yanofsky et al. [O.H. Smith and C. Yanofsky, Methods in Enzymology vol. 5, 794-804 (1962)]. The value obtained was 46 µmole/g-gel, 20 min.

### Example 4

Escherichia coli K-12 YK 3004 (FERM BP-1735) was inoculated into a 500 ml capacity conical flask containing 100 ml of a culture medium having a composition shown in Table 7 and then cultured at 37° C for 13 hours. Moreover, 2 v/v % of said culture was inoculated into 1.5 liter (pH of 7.2, 37° C) of a culture medium having a composition shown in Table 8. While aerating a 3-liter fermenter, the agitation culture was conducted. At the time when the tubidity (OD₆₆₀ₙₘ) of the cultural filtrate reached 15 or so, MgSO₄.7H₂O and FeSO₄.7H₂O were so added that respective final concentrations would be 400 mg/l and 100 mg/l.

**Table 7**

| | |
|---|---|
| K₂HPO₄ | 5.5 g |
| KH₂PO₄ | 1.6 g |
| (NH₄)₂SO₄ | 3 g |
| MgSO₄.7H₂O | 0.1 g |
| Polypeptone | 1 g |
| Yeast extract | 1 g |
| Ampicillin | 50 mg |
| Glucose | 5 g |
| Water | 1 liter |

**Table 8**

| | |
|---|---|
| K₂HPO₄ | 11.0 g |
| KH₂PO₄ | 3.2 g |
| (NH₄)₂SO₄ | 6 g |
| MgSO₄.7H₂O | 0.2 g |
| Polypeptone | 1 g |
| Yeast extract | 1 g |
| FeSO₄.7H₂O | 50 mg |
| Glucose | 10 g |
| Water | 1 liter |

Further, the concentration of a dissolved oxygen in the cultural filtrate was measured by means of a dissolved oxygen electrode. At the time when the glucose concentration reached zero and the dissolved oxygen value of the cultural filtrate began to rise, glucose was so added as to have a concentration of 1 %. At the time when the culturing was continued and an increase of OD₆₆₀ was observed no longer, the culturing was finished and a cell was recovered centrifugally. By using 5 g (wet weight) of the cell there was obtained 11.5 g of immobilized cell of Escherichia coli in accordance with the procedure of Example 3. The tryptophanase activity of this immobilized microbial cell was measured in the following way. Namely, a reaction liquid shown in Table 9 was added to immobilized microbial cell of Escherichia coli and its shaking was conducted at 37° C for one hour. The immobilized microbial cell was filtered and water was added to the filtrate to bring its total amount to one liter. The resultant L-tryptophane was completely dissolved and its amount formed was determined by means of a high-pressure liquid chromatography (LC-5A, a product of Shimadzu Seisakujo). As a result, the tryptophanase activity value of the immobilized microbial cell of Escherichia coli was 0.24 g-tryptophane/g-gel·hr.

**Table 9**

| | |
|---|---|
| Indole | 1.5 g |
| L-serine | 10 g |
| KCl | 0.2 g |
| Pyridoxale-5'-phosphoric acid | 0.5 mg |

Its total amount was brought to be 50 ml (pH of 9.0) by using a 100 mM tris buffer solution.

### Example 5

Brevibacterium flavum JM 233-AB-41 (FERM BP-1498) was inoculated into a 500 ml capacity conical flask containing 100 ml of a culture medium having a composition shown in Table 10 and then cultured at 30° C for 24 hours. Moreover, 2 v/v % of said culture was inoculated into a culture medium (pH of 7.6) having a composition shown in Table 11. While aerating a 3-liter fermenter, the agitation culturing was conducted at 33° C for 20 hours. By using 5 g (wet weight) of the cell collected centrifugally there was obtained 15 g of immobilized microbial cell of Brevibacterium flavum in accordance with the procedure of Example 3. Its aspartase activity was measured in the following way. Namely, a reaction liquid shown in Table 12 was added to immobilized microbial cell of Brevibacterium flavum and its shaking was conducted at 46° C for one hour. An amount of aspartic acid decreased was determined by means of a high-pressure liquid chromatography (LC-5A, a product of Shimazdzu Seisakujo). As a result, the aspartase activity of the immobilized microbial cell of Brevibacterium flavum was 130 µmol/g-gel·hr.

**Table 10**

| | |
|---|---|
| Urea | 4 g |
| (NH₄)₂SO₄ | 0.5 g |
| K₂HPO₄ | 0.5 g |
| MgSO₄.7H₂O | 0.5 g |
| Yeast extract | 1 g |
| Casamino acid | 1 g |
| Biotin | 200 µg |
| Thiamine hydrochloride | 100 µg |
| FeSO₄.7H₂O | 6 mg |
| Water | 1liter |

**Table 11**

| | |
|---|---|
| (NH₄)₂SO₄ | 23 g |
| KH₂PO₄ | 0.5 g |
| K₂HPO₄ | 0.5 g |
| MgSO₄.7H₂O | 0.5 g |
| Yeast extract | 3 g |
| Casamino acid | 3 g |
| Biotin | 200 µg |
| Thiamine hydrochloride | 100 µg |
| FeSO₄.7H₂O | 20 mg |
| MnSO₄.4-6H₂O | 20 mg |
| Water | 1liter |

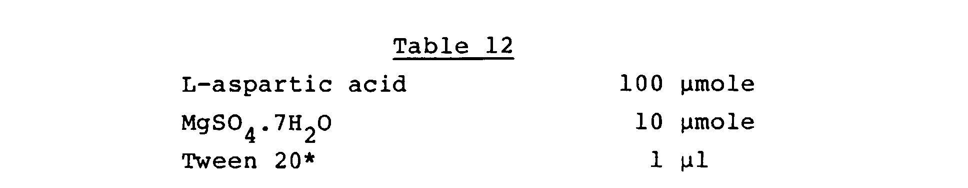

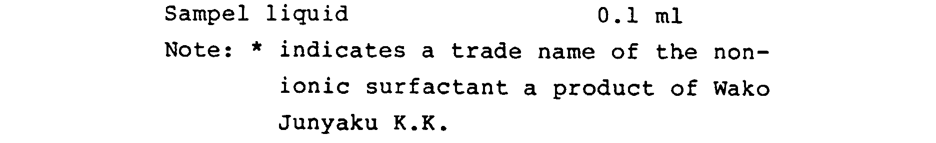

Its total amount was brought to be 1 ml (pH of 7.4) with a 100 mM tris buffer solution.

### Example 6

By taking one gram of each of total six kinds of the immobilized microbial cells of the present invention prepared in Examples 1, 3 and 5 and immobilized microbial cells prepared in each of these Examples except that no crosslinking agent was used this was added to a 200 ml capacity conical flask containing 100 ml of 0.2 M Na₂HPO₃ (pH of 9) and its shaking was conducted at 40° C for 1.5 hours. Shown in Table 13 is the result obtained by determining the weight of the immobilized microbial cells recovered by filtering the same in order to known the degree of swelling.

**Table 13**

| Immobilized microbial cells | Pseudomonas putida | Escherichia coli | Brevibacterium flavum |
|---|---|---|---|
| Immobilized microbial cell of the present invention | 1.2 g | 1.1 g | 1.5 g |
| Immobilized microbial cell using no crosslinking agent | 8.0 g | 10.2 g | 9.8 g |

## Claims

1. A process for the preparation of an immobilized biocatalyst which comprises the following steps:
(i) mixing an aqueous solution of polyuronic acid with microbial cells or products derived therefrom,
(ii) adding dropwise the mixture obtained in the step (i) into an aqueous gelation solution to form a gel of water-insoluble polyuronic acid entrapping microbial cells or products derived therefrom, and
(iii) contacting the gel with a crosslinking agent containing in its molecule at least two of such reactive functional groups as are selected from the group consisting of an amino group and a hydroxyl group, in an aqueous medium in the presence of a condensation agent.

2. The process according to claim 1 wherein said polyuronic acid is an alginic acid, a pectic acid or a mixture thereof.

3. The process according to claim 1 wherein said crosslinking agent is a water-soluble high polymer compound.

4. The process according to claim 1 wherein said crosslinking agent is of at least one kind selected from the group consisting of polyvinyl alcohol, polyalkyleneimine, polyallylamine and polyvinylamine.

5. The process according to claim 1 wherein said condensation agent is a water-soluble carbodiimide.

## Patentansprüche

1. Verfahren zur Herstellung eines immobilisierten Biokatalysators, **gekennzeichnet** durch die folgenden Stufen:
(i) Mischen einer wäßrigen Lösung einer Polyuronsäure mit Mikrobenzellen oder Produkten, die sich davon ableiten;
(ii) tropfenweise Zugabe des bei der Stufe (i) erhaltenen Gemisches in eine wäßrige Gelatine-Lösung unter Bildung eines Gels aus wasserunlöslicher Polyuronsäure, welches die Mikrobenzellen oder die Produkte, die sich davon ableiten, eingeschlossen enthält; und
(iii) Behandlung des Gels mit einem Vernetzungsmittel, das in seinem Molekül mindestens zwei von solchen reaktiven Gruppen enthält, die ausgewählt werden aus der Gruppe bestehend aus einer Aminogruppe und einer Hydroxylgruppe, in einem wäßrigen Medium in Anwesenheit eines Kondensationsmittels.

2. Verfahren nach Anspruch 1, dadurch **gekennzeichnet**, daß die Polyuronsäure eine Alginsäure, eine Pectinsäure oder ein Gemisch davon ist.

3. Verfahren nach Anspruch 1, dadurch **gekennzeichnet**, daß das Vernetzungsmittel eine wasserlösliche Hochpolymerverbindung ist.

4. Verfahren nach Anspruch 1, dadurch **gekennzeichnet**, daß das Vernetzungsmittel von mindestens einer Art, ausgewählt aus der Gruppe bestehend aus Polyvinylalkohol, Polyalkylenimin, Polyallylamin und Polyvinylamin, ist.

5. Verfahren nach Anspruch 1, dadurch **gekennzeichnet** daß das Kondensationsmittel ein wasserlösliches Carbodiimid ist.

## Revendications

1. Procédé pour la préparation d'un biocatalyseur immobilisé qui comprend les étapes suivantes :
(i) mélange d'une solution aqueuse d'acide polyuronique avec des cellules microbiennes ou des produits dérivées de ces cellules,
(ii) addition goutte à goutte du mélange obtenu à l'étape (i) dans une solution aqueuse pour former un gel d'acide polyuronique insoluble dans l'eau en encapsulant des cellules microbiennes ou des produits dérivés de ces cellules, et
(iii) mise en contact du gel avec l'agent réticulant contenant dans sa molécule au moins deux groupes fonctionnels réactifs choisi dans le groupe comportant un groupe amino et un groupe hydroxyle, dans milieu aqueux en présence d'un agent de condensation.

2. Procédé selon la revendication 1, où ledit acide polyuronique est un acide alginique, un acide pectique ou un mélange de ces acides.

3. Procédé selon la revendication 1, où ledit agent de réticulation est un composé haut polymère soluble dans l'eau.

4. Procédé selon la revendication 1, où ledit agent de réticulation est au moins un type choisi dans le groupe comprenant le poly(alcool vinylique), la polyalkylèneimine, la polyallylamine et la polyvinylamine.

5. Procédé selon la revendication 1, où ledit agent de condensation est un carbodiimide soluble dans l'eau.
